# EUROPEAN PATENT SPECIFICATION

(11) **EP 2 322 512 B1**
(45) Date of publication and mention of the grant of the patent: **21.10.2015**
(21) Application number: 10170216.5
(22) Date of filing: 21.07.2010
(51) Int. Cl.: C07D 235/26

(54) **Alkylated benzimidazolone compounds and self-assembled nanostructures generated therefrom**
Alkylierte Benzimidazolonzusammensetzung und daraus hergestellte selbstangeordnete Nanostrukturen
Composants de benzimidazolone d'alkylat et nanostructures auto-assemblées générées à partir de ceux-ci

(30) Priority: 19.10.2009 US 581488; 11.05.2010 US 777329
(43) Date of publication of application: 18.05.2011
(73) Proprietor: Xerox Corporation, Rochester, NY 14644 (US); National Research Council of Canada, Ottawa, Ontario K1A 0R6 (CA)
(72) Inventor: Makeiff, Darren, St. Alberta Alberta T8N 2C1 (CA); Carlini, Rina, Oakville Ontario L6H 0B9 (CA); Fenniri, Hicham, Edmonton Alberta T6R 3C2 (CA)
(74) Representative: Grünecker Patent- und Rechtsanwälte PartG mbB

(56) References cited:
- EP-A2- 2 290 013
- JP-A- 2003 096 056
- JP-A- 2003 238 842
- JP-A- 2003 252 864
- JP-A- 2009 221 266
- US-A1- 2007 012 221
- US-B1- 7 563 318
- DATABASE REGISTRY [Online] CHEMICAL ABSTRACTS SERVICE, COLUMBUS, OHIO, US; 4 August 2008 (2008-08-04), XP002610243, retrieved from STN accession no. 1038266-62-4
- DATABASE REGISTRY [Online] CHEMICAL ABSTRACTS SERVICE, COLUMBUS, OHIO, US; 29 August 2002 (2002-08-29), XP002610244, retrieved from STN accession no. 445410-55-9
- MAURITS H. K. EBBING, MARÍA-JESÚS VILLA, JOSÉ-MARÍA VALPUESTA, PILAR PRADOS, AND JAVIER DE MENDOZA: "Resorcinarenes with 2-benzimidazolone bridges: Self-aggregation, self-assembled dimeric capsules, and guest encapsulation", PNAS, vol. 99, no. 8, 2002, pages 4962-4966, XP002610245,
- SCHWIEBERT K E ET AL: "Engineering the solid state with 2-benzimidaolones", JOURNAL OF THE AMERICAN CHEMICAL SOCIETY, AMERICAN CHEMICAL SOCIETY, US, vol. 118, no. 17, 1 May 1996 (1996-05-01), pages 4018-4029, XP002175323, ISSN: 0002-7863, DOI: DOI:10.1021/JA952836L

## Description

### TECHNICAL FIELD

This disclosure is generally directed to amphiphilic organic compounds with hydrogen-bonding (H-bonding) functionalities that can reversibly self-assemble into well-defined nanostructures. More specifically, the present disclosure relates to amphiphilic alkylated benzimidazolone compounds and self-assembled nanostructures generated therefrom. These nanostructures include a variety of different nanoparticle morphologies, often described as spherical shaped particles, planar sheets, or pseudo one-dimensional structures such as fibrils, ribbons, tapes, tubes, rods, belts, etc.

### BACKGROUND

Recent technology trends in materials science indicate that the use of nanotechnology-enabled components and materials are gaining more appeal due to the enhanced (and sometimes even breakthrough) performance being exhibited. Functional nanomaterials exhibit many unique and often tunable physical and chemical properties that are different than those of their bulk counterparts. Developments have been recently made towards the fabrication of nanomaterials having well defined shape and dimensions involving either "top down" or "bottom up" fabrication strategies. "Top down" approaches involve cutting down larger structures into the desired shape with the desired dimensions (e.g. nanolithography). "Bottom up" strategies involve growing structures of the desired shape and dimensions from smaller building blocks (e.g. self-assembly). The latter is the preferred approach because it is much more efficient and bypasses the need for cost-intensive and energy-intensive fabrication processes.

Molecular self-assembly is a practical "bottom up" approach to arrive at nanostructured materials. In this approach, self-complementary molecules are designed as 'building blocks' with a specific size, shape and at least one functional group, to aggregate in an ordered manner. The resulting ensemble often possesses completely different properties than their smaller building subunits. However, the challenge of this approach is to design the appropriate molecular subunits that can assemble into useful nanostructures in a controlled manner such that the final desired size and shape can be achieved. Consequently, the modular use of hydrogen-bonding molecular building blocks is key to designing novel nanoscale supramolecular structures, non-covalent polymers, organogelators, and liquid crystals, that have useful properties for developing advanced functional materials such as for example adhesives, self-healing coatings, as well as many others.

Cyclic urea compounds that contain the benzimidazolone (BZI) functional group can self-assemble into hydrogen-bonded (H-bonded) dimer structures in the solid state resembling tapes or ribbons. These tape-like structures can vary in size and morphology depending on the type and position of functional substituents present on the benzimidazolones.

F. H. Herbstein et al., "Crystal and Molecular Structure of 1,3-dihydro-2H-benzimidazol-2-one (the solid state tautomer of 2-hydroxybenzimidazolone)", Z. Kristallogr, vol. 173, p. 249-256 (1985), describes the crystal structure of 1,3-dihydro-2*H*-benzimidazol-2-one. Ribbons of antiparallel keto-tautomer molecules linked by a zigzag set of (N-H)---(O=C) donor-acceptor pairs of hydrogen bonds are formed. The ribbons are planar and lie in a herring-bone pattern.

G. M. Whitesides et al., "Engineering the Solid State with 2-Benzimidazolones", J. Am. Chem. Soc., vol. 118, p. 4018-4029 (1996), describe the solid state structures of six 2-benzimidazolone derivatives, disubstituted in the 4 and 5 positions on the benzene ring. 2-Benzimidazolones having either methyl, chlorine, and bromine atoms at the 4 and 5 positions form tapes, which pack differently than 2-benzimidazolones with hydrogens in the same positions. In contrast, three-dimensional networks were formed from 2-benzimidazolones having fluorine or iodine substituents at both 4 and 5 positions.

E. F. Paulus, "Molecular and crystal structure of C. I. Pigment Red 208, 12514, n-Butyl-2-{2-oxo-2,3-dihydro-5-benzimidazolyl)-carbamoyl]-naphthylidenhydrazino}-benzoate (PV-Rot HF2B)", Z. Kristal., vol. 160, p. 235-243 (1982) describes the crystal structure of azo-benzimidazolone Pigment Red 208. The pigment molecules are organized into tape-like structures. Each benzimidazolone group of the pigment molecules interacts with only one other benzimidazolone group from another neighboring pigment molecule to form a dimer assembly *via* a 2-point H-bonding interaction involving a carbonyl (C=O) acceptor and -(N-H) donor from each monomeric subunit. Each dimer is then further bound to two other dimers via two single-point H-bonding interactions between the benzimidazolone -(N-H) donor group and the 2-oxo-3-naphthylamido carbonyl group acceptor. The tapes have lipophilic edges and are further organized into layers in the crystal structure.

K. Hunger et al, "Über die Moleküund Kristallstruktur gelber Mono-"azo"-Pigmente", Farbe & Lack, vol. 88, p. 453-458 (1982) describes the crystal structure of a yellow azo-benzimidazolone pigment. The pigment molecules are organized into tape-like structures. Each benzimidazolone group of one pigment molecule subunit interacts with only one benzimidazolone group from another neighboring pigment molecule subunit, to form a dimer assembly *via* a 2-point interaction involving a carbonyl (C=O) acceptor and -(N-H) donor from each half. Each dimer is then further bound to two other dimers via two single-point H-bonding interactions between the benzimidazolone -(N-H) donor and the acetoamido carbonyl group acceptor. The tapes are packed into a zig-zag type arrangement in the crystal structure.

J. van de Streek et al, "Structures of six industrial benzimidazolone pigments from laboratory powder diffraction data", Acta Cryst., B65, p. 200-211 (2009) describes the crystal structures of six industrially produced benzimidazolone pigments modeled from X-ray powder diffraction data. The six industrial pigments exhibited five different tape-like hydrogen-bonded motifs.

Although hydrogen-bonded tape or ribbon structures have been observed in solid state X-ray crystal structures, it has not yet been demonstrated that benzimidazolones form "free-standing" nanostructures in solutions or dispersions. To our knowledge, the only microscopy studies that have been performed on self-assembled aggregates of benzimidazolone derivatives were by J. de Mendoza *et al*.

J. de Mendoza et al, "Resorcinarenes with 2-benzimdazolone bridges: self-aggregation, self-assembled dimeric capsules, and guest complexation", Proc. Natl. Acad. Sci. USA, 99, 4962-4966 (2002) describes the synthesis and self-assembly behavior tetra-2-benzimidazolone functionalized resorcinarene compounds having various pendant alkyl groups. Self-organized structures such as micron-sized vesicles and long fibers were formed depending on the nature and length of the four pendant carbon chains attached at the bottom of each resorcinarene platform. Solvophobic effects, van der Waals interactions, and the packing of alkyl chains drive the formation of these higher order supramolecular assemblies from the capsules, as compared to the extensive hydrogen-bonded chains involving the benzimidazolone functional groups for the compounds of the present invention.

There remains a need for new and improved nanotechnology-enabled components and materials, particularly those having self-complementary functional groups which can self-assemble readily by a "bottom up" fabrication strategy to produce well-defined nanostructures and potentially higher-order network structures, that can be useful and desirable properties in developing functional materials.

### SUMMARY

The present disclosure addresses these and other needs, by providing alkylated benzimidazolone compounds and self-assembled nanostructures formed from alkylated benzimidazolone compounds.

The present disclosure provides an alkylated benzimidazolone compound selected from the group consisting of the following compounds:

| | | |
|---|---|---|
| | 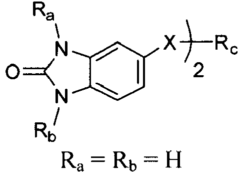 | |

| | **Group X** | **R_{c}** |
|---|---|---|
| **1** | 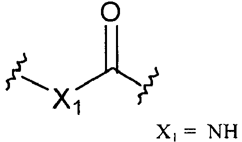 | 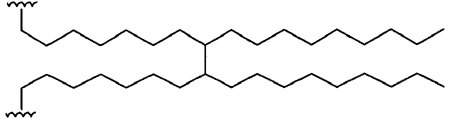 |
| **2** | 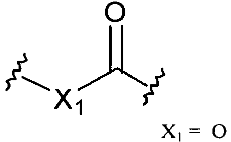 | 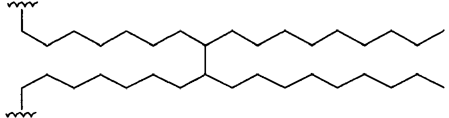 |
| **3** | 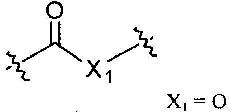 | 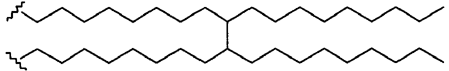 |
| **4** | 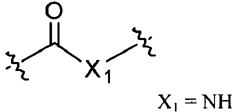 | 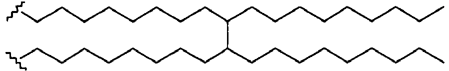 |

and mixtures thereof.

The present disclosure further provides a nanostructure comprising molecules of said compound non-covalently bound to each other.

The present disclosure further provides a marking material composition comprising said nanostructure, wherein the marking material is an ink, a toner, a developer, a paint, or a coating.

The present disclosure further provides an organogel composition comprising said nanostructure.

Preferred embodiments of the present disclosure are set forth in the dependent claims.

### DETAILED DESCRIPTION OF EXEMPLARY EMBODIMENTS

Terms, when used in this application, have their common meaning unless otherwise stated.

The term "nanostructure" shall refer to a physical structure (e.g. a particle or the like), which, in at least one dimension, such as the smallest dimension, has a size ranging from about 1 or about 10 or about 20 to about 100 or to about 200 or to about 500 nm, such as between about 10 to about 300 nm, and which has a largest dimension that is desirably less than about 5000 nm in size, such as less than about 2000 nm in size, or less than about 1000 nanometers in size.

The term "1D structure" shall refer to a structure having a significantly larger length than height or width (or diameter). The aspect ratio, defined as length divided by the width can be at least about 5 or at least about 10, such as about 100-500. These 1D structures can thus take the form of strings (which in the case of being electrically conductive may be referred to as wires), tapes, or the like.

The term "2D structure" shall refer to a flat, planar structure having length and width that are comparable in size, but no depth (or negligible depth). The aspect ratio can be at most about 5, such as about 2, or about 1. "2D Structures" may be either porous or non porous sheet structures (e.g. a film or wafer).

The term "3D structure" shall refer to a structure that possesses the dimensions of length, width, and height that are comparable and appreciable in relative size. In the context of this disclosure, the term "3D structure" refers to a higher order arrangement of smaller (more elementary) nanostructures; i.e. 1D structures. 3D structures may include porous networks like, for example a gel network, or even more highly ordered, less porous networks such as liquid crystals.

The term "nanofibril" shall refer to a 1D structure resembling a long slender filament or fiber with diameter desirably less than about 100 nm size, such as less than about 50 nm in size, or less than about 20 nm in size. The length of the nanofibril can range from about 20 nm up to about 5000 nm or larger.

The term "nanofiber" shall refer to a 1D structure resembling a thick filament or fiber with a diameter desirably less than about 200 nm in size, or less than about 100 nm, or about 50 nm in size. "Nanofibers" in the context of this invention may consist of a single structural element or may be composed of more than one structural element, such as a bundle of smaller "nanofibrils".

Embodiments of the present disclosure provide alkylated benzimidazolone compounds and self-assembled nanostructures formed from alkylated and substituted benzimidazolone compounds.

The alkylated benzimidazolone compounds have the function of self-assembling into larger structures, either alone or in combination with other materials. For example, the compounds can be used to self-assemble with colorant molecules to form a nanoscale pigment particle composition, such as disclosed in U.S. Patent Application No. 12/405,079 filed March 16, 2009. The alkylated benzimidazolone compounds may thus limit the extent of primary particle aggregation and growth, so as to produce predominantly nanoscale particles.

The alkylated benzimidazolone compounds have a hydrocarbon moiety that provides sufficient steric bulk to enable the function of the compound to regulate particle size of the aggregated structures.

The types of non-covalent chemical bonding that can occur between separate molecules of the alkylated benzimidazolone compounds, or between the alkylated benzimidazolone compounds and other compounds, are, for example, van der Waals forces, ionic or coordination bonding, H-bonding, and/or aromatic pi-stacking bonding. In embodiments, the non-covalent bonding is predominately H-bonding and van der Waals' forces, but can include aromatic pi-stacking bonding as additional or alternative types of non-covalent bonding between the respective molecules.

The organic nanostructures from the alkylated BZI compounds described herein can be prepared, for example, by homogeneously mixing a self-assembling, alkylated BZI derivative having the above formula with a polar or nonpolar liquid under conditions sufficient to effect the extent of dissolution and self-assembly, usually by heating followed by subsequent cooling and aging for a given period of time to allow the desired nanostructures to fully mature. Mixing of the components may be conducted at temperatures ranging between room temperature and the boiling point of the liquid. The self-assembling, alkylated BZI compound may be added in the form of powder particles, which may completely dissolve in the liquid to form a clear solution or may only partially dissolve to form a dispersion. Alternatively the self-assembling, alkylated BZI compound may be added as a solution dissolved in a suitable solvent including both polar and nonpolar liquids. This liquid that the alkylated BZI compound is dissolved in may be the same as the liquid it is being added to, or may be a different liquid. In addition, the liquid to which the solution of alkylated BZI compound is being added to may be a good or poor solvent for the alkylated BZI compound and resulting self-assembled nanostructures. The nanostructure compositions of the present invention may also be formed, for example, at elevated temperatures by dissolving or dispersing the self-assembling alkylated BZI compound in the liquid at elevated temperatures, and thereafter cooling the resulting solution to a lower temperature, whereby a colloid solution or dispersion of nanostructured aggregates forms while aging for a suitable period of time.

According to the present disclosure, the self-assembling alkylated BZI compound may be present in a wide range. Preferred is a range of about 0.05% to 20% by weight based upon the liquid of the composition, more preferably 0.075 to 10%, and even more preferably 0.1 to 1.5 to 2.0%. The properties of the compositions containing the nanostructures may be controlled depending on the kind and amount of alkylated BZI compound added. A suitable amount of alkylated BZI compound may be readily determined by routine experimentation and will vary with the desired physical property of the composition and other components therein. As is understood by those skilled in the art, a lower amount of alkylated BZI compound often makes the compositions more desirable, inasmuch as the non-assembled, individual alkylated BZI molecules may often demonstrate chemical and physical properties that are different from the end use properties of the compositions containing self-assembled nanostructures from alkylated benzimidazolone compounds.

More than one self-assembling BZI compound may be utilized to form nanostructures in a particular liquid. For example, a mixture of two different isomers or homologues of a particular alkylated BZI compound (e.g., different linkages, organic substituents, etc.) may be used.

When preparing the self-assembled nanostructures, the requisite amount of alkylated BZI is mixed with the liquid and the materials are blended, for example under ambient conditions of temperature and pressure. Different temperatures and pressures may be utilized in the mixing process where, for example, loss of vapors, in the case of a low-boiling liquid hydrocarbon, is to be avoided (use lower temperatures and/or higher pressures) or when easier mixing, in the case of higher-boiling liquids, is to be obtained (use higher temperatures and/or lower pressures).

The components may be mixed by any means such as stirring, shaking, or passing the mixtures through a homogenizer, or subjecting to ultrasonic waves to produce a homogeneous composition. Regardless of the method of blending, self-assembled nanostructures are produced as a result of obtaining a solution or dispersion of the alkylated BZI compound in the liquid.

The compositions of self assembled nanostructures of the present disclosure, once formed, may be contained in liquid or in solid form upon evaporation of the liquid. Liquid compositions may vary, and consist of clear or turbid colloidal solutions, opaque dispersions, settled precipitates, clear viscous (supramolecular)polymer solutions, or thick gels. The viscosity of liquid compositions of the nanostructures varies from thin, pourable type to a shape retaining material (i.e., a gel). The resulting nanostructures may be robust, individually dispersed, or highly cohesive, and are stable in storage for variable periods (depending on the alkylated BZI compound, its concentration, the liquid, and the temperature of storage), thermally reversible, and are sheer stress thinnable.

The self-assembled nanostructures made from the alkylated benzimidazolone compounds described herein generally comprise the alkylated benzimidazolone compounds in a major, predominant, substantial, or entire amount of the solid form of the nanostructure. That is, in embodiments, the solid portion of the nanostructures (not including any solvent or liquid carrier that may be included) comprises, consists essentially of, or consists of the alkylated benzimidazolone compounds. Of course, two or more different alkylated benzimidazolone compounds can be included, as desired. Thus, in embodiments, the nanostructures do not contain other hydrogen-bonding materials such as steric stabilizers, and do not correspond to nanoparticles that may be formed by association of the alkylated benzimidazolone compounds with pigment particles.

However, in other embodiments, the nanostructure may comprise one or more additives, such as to provide desired properties to the nanostructure. For example, the additives may provide such properties as hardness, rigidity, porosity, color, or the like to the nanostructure. Such additives in embodiments do not hydrogen bond to the alkylated benzimidazolone compounds in the nanostructure. Instead, in these embodiments, the additives can be covalently or ionically bound to the nanostructure, or they can be mixed, dispersed, or the like in the nanostructure.

A number of characterization methods are useful for detecting and characterizing self-assembled nanostructures from alkylated BZI compounds. The simplest test is to observe any changes in viscosity (rheology) of the liquid containing the alkyl BZI compound relative to the neat liquid alone. A highly viscous fluid or jelly-like material strongly suggests the formation of nanostructured supramolecular aggregates (i.e., supramolecular polymers or gels). If the mixture does not flow under the influence of gravity upon inversion of the sample vial, then the mixture is considered to be a gel. The increase in viscosity and gelation of liquids is known to occur due to the presence and entanglement of long, 1D aggregates.

Microscopy techniques such as optical light microscopy, scanning electron microscopy (SEM), transmission electron microscopy (TEM), atomic force microscopy (AFM)/scanning probe microscopy (SPM), and fluorescence microscopy are useful for determining the size and morphology of the nano (and micro)structures formed from alkylated BZI compounds. Samples are typically prepared by depositing a drop of the liquid composition containing the nanostructures onto an appropriate sample substrate such as a carbon film coated copper mesh TEM grid, removing the excess liquid by blotting with filter paper, and then allowing to dry prior to analysis. Dynamic light scattering is also useful for detecting the presence of particles between 1 nm and 1 µm in size, measuring the size/size distribution of the dispersed particles. Rheometry is useful for determining the viscoelastic properties and thermal phase transitions for compositions of the self-assembled nanostructures. X-ray diffraction is useful for characterizing the structure of the self-assembled nanostructures size as phase identification, crystallinity, phase transitions, crystal structure refinement and determination, and size and strain broadening of crystallite nanostructures. NMR spectroscopy is useful in detecting the formation intermolecular noncovalent interactions stabilizing the nanostructures, their diffusion properties, as well as phase transitions. UV-Vis can be used for detecting the presence of nanostructures as well as confirming the presence of intermolecular pi-stacking interactions. FT-IR spectroscopy is also useful for the detection of hydrogen-bonding interactions involved in stabilizing the self-assembled nanostructures. Differential Scanning Calorimetry (DSC) is another useful characterization technique, which enables the identification of thermal phase transitions within the compositions containing the nanostructures.

As disclosed in U.S. Patent Application No. 12/405,079, the alkylated benzimidazolone compounds can be used for making nanoscale particles of azo-benzimidazolone organic pigments, by using a bottom-up assembly synthetic approach that makes use of the alkylated benzimidazolone compounds as amphiphilic surface auxiliaries for controlling the particle size, morphology, dispersion properties and even coloristic properties of the resulting nanopigments. The procedures disclosed therein can be used to make other suitable nanopigments and nanocolorants.

The alkylated benzimidazolone compounds, and self-assembled structures made from those compounds, can be used in a wide variety of applications. For example, the alkylated benzimidazolone compounds can be used as organogelators in the formation of organogels, which may then be used as thickening agents for numerous products such as paints, inks, coatings, lubricants, adhesives, personal care products, pharmaceutical and dermatological gels, and even in certain food products, or they can be used in tissue engineering, biomineralization (as templates), catalysis, gel-based scaffolds for energy transfer and light harvesting, and the like. The alkylated benzimidazolone compounds can also be used in the formation of novel hydrogen bonded liquid crystal materials, where the liquid crystal material can comprise the alkylated benzimidazolone compounds disclosed herein themselves, or in combination with another complementary H-bonding molecules or polymers with pendant complementary H-bonding groups.

The alkylated benzimidazolone compounds, and self-assembled structures made from those compounds, can also be used in combination with coloring agents in a variety of ink and coating compositions, such as in liquid (aqueous or non-aqueous) printing ink vehicles, including inks used in conventional pens, markers and the like, liquid inkjet ink compositions, solid or phase change ink compositions, paints and automotive coatings, and the like. For example, the compounds can be formulated into a variety of ink vehicles, including solid and phase-change inks with melt temperatures of about 60 to about 130°C, solvent-based liquid inks or radiation-curable such as UV-curable liquid inks, and even aqueous inks.

In addition to ink compositions, the compounds can be used in combination with coloring agents in a variety of other applications, such as for paints, resins and plastics, lenses, optical filters, and the like according to applications thereof. By way of example only, the compounds can be used for toner compositions, which include polymer particles and pigment particles, along with other compounds that are formed into toner particles and optionally treated with internal or external additives such as flow aids, charge control agents, charge-enhancing agents, filler particles, radiation-curable agents or particles, surface release agents, and the like. Toner compositions can be prepared by a number of known methods including extrusion melt blending of the toner resin particles, pigment particles and other colorants and other optional additives, followed by mechanical comminution and classification. Other methods include those well known in the art such as spray drying, melt dispersion, extrusion processing, dispersion polymerization, and suspension polymerization. Further, the toner compositions can be prepared by emulsion/aggregation/coalescence processes, as disclosed in references U.S. Patents Nos. 5,290,654, 5,278,020, 5,308,734, 5,370,963, 5,344,738, 5,403,693, 5,418,108, 5,364,729, 5,346,797, 7,547,499, 7,524,599, 7,442,740, 7,429,443, 7,425,398, 7,419,753, 7,402,371, 7,358,022, 7,335,453, and 7,312,011. The toner particles can in turn be mixed with carrier particles to form developer compositions. The toner and developer compositions can be used in a variety of electrophotographic printing systems.

### EXAMPLE

All proportions in the following example are by weight unless otherwise indicated.

### Synthesis of bis-[5,5-(9',10'-dinonyloctadecanamido)-2-benzimidazolone] (compound #1):

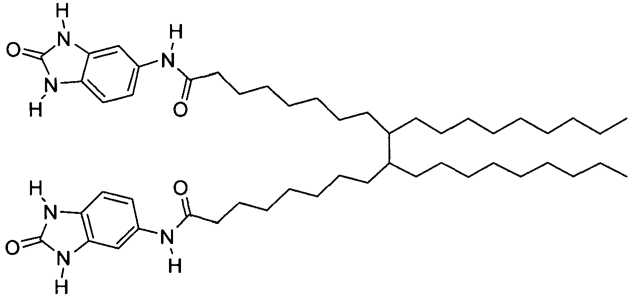

### Step I - Synthesis of 9,10-dinonyloctadecanoyl dichloride:

9,10-dinonyloctadecanoic acid (Pripol 1006, 3.44g, 6.07 mmol) and dry THF (50mL) are added to a 250mL round bottom flask under an inert atmosphere and cooled to 0 °C. Oxalyl chloride (3.20mL, 36.7 mmol) is added slowly, dropwise, followed by DMF (0.140mL, 1.81 mmol). The mixture is then slowly allowed to warm to room temperature and is stirred for 3.5 h. before the solvent is removed by rotary evaporation and dried *in* vacuo to give a pale yellow oil. The diacid chloride compound thus obtained was used in the next step without further purification.

### Step II - Synthesis of bis-[5,5-(9',10'-dinonyloctadecanamido)-2-benzimidazolone]:

5-Aminobenzimidazolone (1.92g, 12.8 mmol), triethylamine (2.5mL, 1789 mmol) and dry *N*-methylpyrrolidinone (NMP, 20mL) are mixed in a 100mL round bottom flask under an inert atmosphere. To this solution, a second solution of 9,10-dinonyloctadecanoyl dichloride from Step I dissolved in dry THF (50mL) is slowly added. After stirring overnight, deionized water (50mL) is added to the beige suspension and the solid was collected by vacuum filtration and washed with deionized water to give bis-[5,5-(9',10'-dinonyloctadecanamido)-2-benzimidazolone] as a beige powder (4.87g). The product is identified by ¹H and ¹³C NMR spectroscopy and ESI-MS and is of satisfactory purity.

## Claims

1. An alkylated benzimidazolone compound selected from the group consisting of the following compounds:
| | |
|---|---|
| 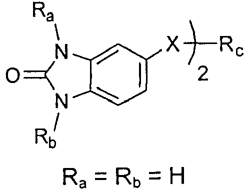 | |
| **Group X** | **R_{c}** |
|---|---|
| 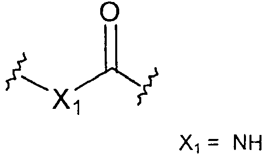 | 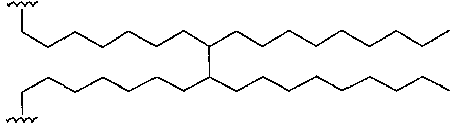 |
| 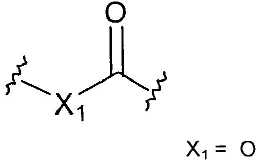 | 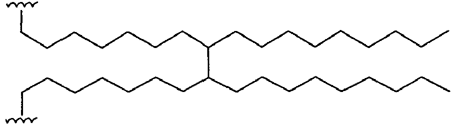 |
| 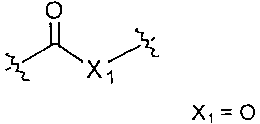 | 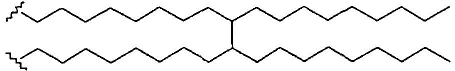 |
| 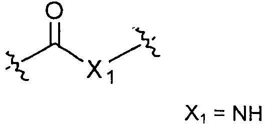 | 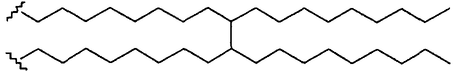 |
and mixtures thereof.

2. A nanostructure comprising molecules of the compound according to claim 1 non-covalently bound to each other.

3. The nanostructure of claim 2, wherein the non-covalent bonding is predominately through hydrogen-bonding, aromatic pi-pi interactions, and van der Waals' forces.

4. The nanostructure of claim 2, wherein the nanostructure is formed by homogeneously mixing the compound with a polar or nonpolar liquid under conditions to effect dissolution and self-assembly of the compound.

5. The nanostructure of claim 2, wherein the nanostructure is a one-dimensional structure in the form of a nanofibril or nanofiber.

6. The nanostructure of claim 2, wherein the nanostructure is a two-dimensional structure.

7. The nanostructure of claim 2, wherein the nanostructure is a three-dimensional structure in the form of a non-covalent gelator network or gel.

8. The nanostructure of claim 2, wherein the nanostructure has, in at least one dimension, a size ranging from 1 to 500 nm, and has a largest dimension of up to 5000 nm.

9. A marking material composition comprising the nanostructure of claim 2, wherein the marking material is an ink, a toner, a developer, a paint, or a coating.

10. An organogel composition comprising the nanostructure of claim 2.

## Patentansprüche

1. Alkylierte Benzimidazolonverbindung ausgewählt aus der Gruppe bestehend aus den folgenden Verbindungen:
| | |
|---|---|
| 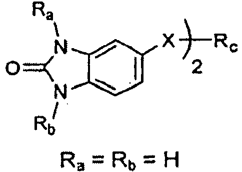 | |
| **Gruppe X** | **R_{c}** |
|---|---|
| 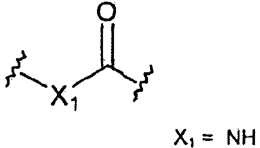 | 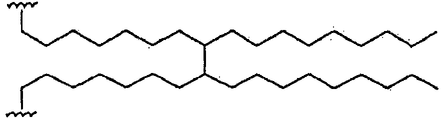 |
| 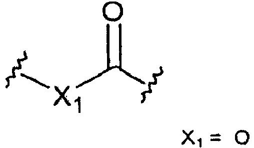 | 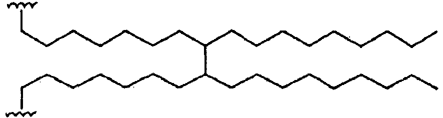 |
| 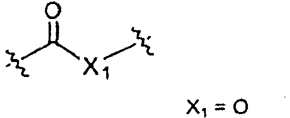 | 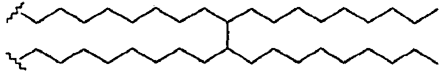 |
| 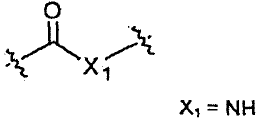 | 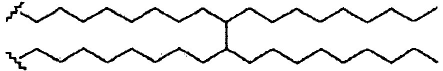 |
und Mischungen davon.

2. Nanostruktur umfassend Moleküle der Verbindung nach Anspruch 1, die nicht-kovalent aneinander gebunden sind.

3. Nanostruktur nach Anspruch 2, wobei die nicht-kovalente Bindung vorwiegend durch Wasserstoffbindung, aromatische pi-pi-Wechselwirkungen und van-der-Waals-Kräfte erfolgt.

4. Nanostruktur nach Anspruch 2, wobei die Nanostruktur durch homogenes Mischen der Verbindung mit einer polaren oder unpolaren Flüssigkeit unter Bedingungen zum Bewirken einer Auflösung und Selbstorganisation der Verbindung gebildet wird.

5. Nanostruktur nach Anspruch 2, wobei die Nanostruktur eine eindimensionale Struktur in Form einer Nanofibrille oder Nanofaser ist.

6. Nanostruktur nach Anspruch 2, wobei die Nanostruktur eine zweidimensionale Struktur ist.

7. Nanostruktur nach Anspruch 2, wobei die Nanostruktur eine dreidimensionale Struktur in Form eines nicht-kovalenten Geliermittelnetzwerks oder Gels ist.

8. Nanostruktur nach Anspruch 2, wobei die Nanostruktur in wenigstens einer Dimension eine Größe im Bereich von 1 bis 500 nm aufweist und eine größte Dimension von bis zu 5000 nm aufweist.

9. Markierungsmaterialzusammensetzung umfassend die Nanostruktur nach Anspruch 2, wobei das Markierungsmaterial eine Tinte, ein Toner, ein Entwickler, ein Anstrichmittel oder ein Überzug ist.

10. Organogelzusammensetzung umfassend die Nanostruktur nach Anspruch 2.

## Revendications

1. Composé benzimidazolone alkylé choisi dans le groupe constitué par les composés suivants :
| | |
|---|---|
| 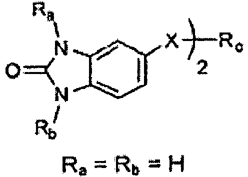 | |
| Groupe X | **R_{c}** |
|---|---|
| 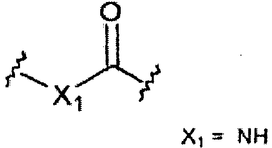 | 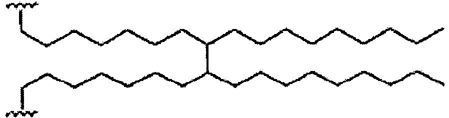 |
| 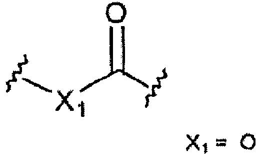 | 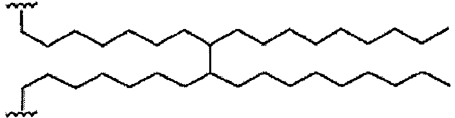 |
| 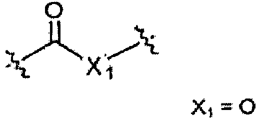 | 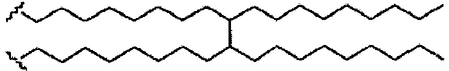 |
| 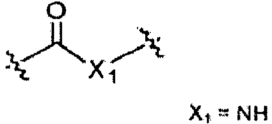 | 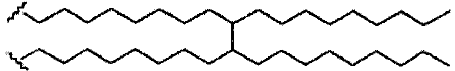 |
et les mélanges de ceux-ci.

2. Nanostructure comprenant des molécules du composé selon la revendication 1 liées de manière non covalente les unes aux autres.

3. Nanostructure selon la revendication 2, dans laquelle la liaison non covalente se fait de manière prédominante par une liaison hydrogène, des interactions pi-pi aromatiques et des forces de van der Waals.

4. Nanostructure selon la revendication 2, dans laquelle la nanostructure est formée par le mélange homogène du composé avec un liquide polaire ou non polaire dans des conditions pour effectuer une dissolution et un auto-assemblage du composé.

5. Nanostructure selon la revendication 2, dans laquelle la nanostructure est une structure unidimensionnelle sous la forme d'une nanofibrille ou d'une nanofibre.

6. Nanostructure selon la revendication 2, dans laquelle la nanostructure est une structure bidimensionnelle.

7. Nanostructure selon la revendication 2, dans laquelle la nanostructure est une structure tridimensionnelle sous la forme d'un réseau de gélifiant non-covalent ou d'un gel.

8. Nanostructure selon la revendication 2, dans laquelle la nanostructure présente, dans au moins une dimension, une taille allant de 1 à 500 nm, et présente une dimension plus grande allant jusqu'à 5 000 nm.

9. Composition de matériau de marquage comprenant la nanostructure selon la revendication 2, dans laquelle le matériau de marquage est une encre, un toner, un révélateur, une peinture ou un revêtement.

10. Composition d'organogel comprenant la nanostructure selon la revendication 2.
